# EUROPEAN PATENT APPLICATION

(11) **EP 4 670 514 A1**
(43) Date of publication of application: **31.12.2025**
(21) Application number: 24760391.3
(22) Date of filing: 21.02.2024
(51) Int. Cl.: A23L 33/135, A23K 10/16, A23K 20/189, A61K 9/14, A61K 35/74, A61K 38/45, A61P 3/04, A61P 3/06, A61P 3/10, A61P 43/00, C12N 1/20, C12N 9/56

(54) **ADVANCED GLYCATION ENDPRODUCT PRODUCTION INHIBITOR**

(30) Priority: 22.02.2023 JP 2023025920
(71) Applicant: Japan Bio Science Laboratory Co., Ltd., Osaka 569-0832 (JP); Josho Gakuen Educational Foundation, Osaka 535-8585 (JP)
(72) Inventor: FUJITA, Mitsugu, Kure-shi, Hiroshima 737-0112 (JP); TAKAOKA, Shinsaku, Osaka-shi, Osaka 553-0003 (JP); INOUE, Kenichi, Osaka-shi, Osaka 553-0003 (JP); NOZAKI, Koji, Kunisaki-shi, Oita 873-0212 (JP); YAMAGUCHI, Moe, Osaka-shi, Osaka 553-0003 (JP)
(74) Representative: Schlich
(86) International application number: PCT/JP2024/006164
(87) International publication number: WO 2024/177093

(57) **Abstract**

An advanced glycation endproduct production inhibitor according to the present invention contains a Bacillus natto culture extract as an active ingredient. According to the present invention, since a Bacillus natto culture extract derived from a food material is contained as a main ingredient, it is possible to safely ingest the advanced glycation endproduct production inhibitor without worrying about side effects. In addition, mass production is possible and distribution to the market can also be easily achieved.

## Description

### Technical Field

The present invention relates to an advanced glycation endproduct (AGE) production inhibitor.

### Background Art

The incidence of type 2 diabetes has been steadily increasing in recent years, and its complications (diseases that induce vascular disorders such as nephropathy, retinopathy, and peripheral neuropathy) are major factors in shortening healthy life expectancy. If such complications are left untreated, they can become fatal, and, for example, nephropathy may lead to artificial dialysis, retinopathy may lead to blindness, and neuropathy may lead to vascular disorders such as silent myocardial infarction and gangrene.

Further, it has been reported that "AGEs" (Advanced Glycation Endproducts) are involved in kidney damage in type 2 diabetes (e.g., Non-Patent Documents 1 to 5), and many studies have been conducted on a relationship between AGEs and the onset and progression of kidney damage. AGEs are also known as "advanced glycation endproducts" or "advanced glycation end products", and refer to a general term for modified proteins formed by oxidative/nonoxidative reactions that begin with a nonenzymatic reaction between proteins and sugars (glycation). The series of reactions involved in the production of AGEs is also known as the Maillard reaction.

It is conceivable that an increase in the amount of AGEs in type 2 diabetes may be related to increased oxidative stress caused by metabolic disorders such as hypertension, hyperlipidemia, obesity, and hyperinsulinemia as well as hyperglycemia.

For this reason, there is a need to establish a technique that can suppress the production of AGEs in vivo and inhibit the onset and progression of type 2 diabetes described above and the like.

### Related Art Documents

### Non-Patent Documents

Non-Patent Document 1: Kang J et al., Acta Diabetol. 2005 Jun; 42(2): 110-6
Non-Patent Document 2: Miyata T et al., Kidney Int. 1998 Feb; 53(2): 416-22
Non-Patent Document 3: Friedman EA, Nephrol Dial Transplant. 1999; 14 Suppl 3: 1-9
Non-Patent Document 4: Christensen EI et al., Nat Rev Mol Cell Biol. 2002, Apr; 3(4): 256-66
Non-Patent Document 5: Gugliucci A et al., Diabetologia. 1996 Feb; 39(2): 149-60

### Summary of Invention

### Problem to be Solved by the Invention

The present invention was made to address the above problems, and it is an object thereof to provide an advanced glycation endproduct production inhibitor that can effectively suppress the production of AGEs in vivo and is highly safe.

### Means for Solving the Problem

The present invention provides an advanced glycation endproduct production inhibitor comprising a Bacillus natto culture extract as an active ingredient.

In one embodiment, the Bacillus natto culture extract contains nattokinase.

In one embodiment, the Bacillus natto culture extract is in the form of a dry powder.

In one embodiment, a content of vitamin K2 contained in the Bacillus natto culture extract is 1 µg or less per gram of a dry mass of the Bacillus natto culture extract.

In one embodiment, the Bacillus natto culture extract has a nattokinase activity of 100 FU/g to 80,000 FU/g.

In one embodiment, the advanced glycation endproduct production inhibitor of the present invention is an oral formulation.

The present invention also provides a method for inhibiting an advanced glycation endproduct production, comprising administering a Bacillus natto culture extract as an active ingredient to an organism.

In one embodiment, the step of administering to the organism is done orally.

The present invention also provides use of compositions containing a Bacillus natto culture extract as an active ingredient to inhibit an advanced glycation endproduct production.

### Effects of the Invention

According to the present invention, since a Bacillus natto culture extract derived from a food material is contained as a main ingredient, it is possible to safely ingest an advanced glycation endproduct production inhibitor without worrying about side effects. In addition, mass production is possible and distribution to the market can be easily achieved.

### Brief Description of Drawings

FIG. 1 is a diagram showing an experiment design carried out in Example 3.
FIG. 2 is a photograph showing a surface of a thin section of a tissue preparation in a histopathological evaluation of renal nephrons carried out in Example 3.
FIG. 3 is a graph obtained by scoring a glycation state of renal tubules based on epithelial cell vacuolation and positive PAS staining in the histopathological evaluation of the renal nephrons carried out in Example 3.
FIG. 4 is a graph showing the result of measurement of the concentration of plasma advanced glycation endproducts (AGEs) carried out in Example 3.

### Description of Embodiments

The advanced glycation endproduct production inhibitor according to the present invention contains a Bacillus natto culture extract as an active ingredient.

The term "advanced glycation endproducts (may be referred to as AGEs hereinafter)" as used herein refers to a general term for modified proteins formed through oxidative/nonoxidative reactions that begin with a nonenzymatic reaction between proteins and sugars (glycation). Furthermore, the term "advanced glycation endproduct production inhibition" as used herein includes both, for example, inhibition of an increase in the amount of AGEs produced through the Maillard reaction in vivo or in vitro to keep it at a substantially constant level, and inhibition of the production of the AGEs to reduce the amount of AGEs produced.

The Bacillus natto culture extract is an extract obtained from a culture medium of Bacillus natto (representatively, Bacillus subtilis natto), which is a type of Bacillus subtilis. The Bacillus natto may be any microorganisms capable of producing nattokinase, which will be described later. The Bacillus natto may be Bacillus natto separated from commercially available natto.

The Bacillus natto culture extract may be in any form such as a dry powder, a liquid, or a paste. It is preferable that the Bacillus natto culture extract is in the form of dry powder due to being easy to handle and readily available as a commercial product, and the like.

A Bacillus natto culture extract, in which natto odor, which is a strong odor peculiar to natto, has been reduced or removed, is suitably used because more people can ingest such a Bacillus natto culture extract with ease. For example, it is preferable to remove certain impurities from the Bacillus natto culture extract in advance in order to reduce or remove such natto odor. Specifically, impurities having a molecular weight of preferably 1000 or less, or more preferably 20,000 or less have been removed from the Bacillus natto culture extract.

The Bacillus natto culture extract contains nattokinase as a main ingredient.

Nattokinase (NK) is a substance with fibrinolytic activity found in a viscous substance of natto, and is a serine protease belonging to the subtilisin family consisting of 275 amino acids. It is known that nattokinase has a high thrombolytic effect in vivo in an in vivo experimental system, and has the effect of improving a peripheral blood flow, an inhibitory effect on platelet aggregation, a hypotensive action for hypertension, an immune enhancing effect, and the like. Also, it is known that, for example, in the in vitro experimental system, nattokinase has a higher fibrinolytic activity than plasmin, degrades and inactivates PAI-1, and increases tissue plasminogen activator (tPA).

The Bacillus natto culture extract has a certain nattokinase activity. The nattokinase activity is expressed in FU (Fibrin degradation Units) per gram, and can be detected by checking whether or not lytic plaques are formed on a fibrin plate according to the method described in, for example, Experientia vol. 43, p. 1110 (1987).

The Bacillus natto culture extract according to the present invention preferably has a nattokinase activity of 100 FU/g to 80,000 FU/gram, and more preferably 4000 FU/g to 80,000 FU/g. If the nattokinase activity of the Bacillus natto culture extract is below 100 FU/g, a contradiction may arise in that a larger amount of the Bacillus natto culture extract is required to obtain a desired effect of efficiently inhibiting the production of the advanced glycation endproducts.

Note that, usually, the nattokinase activity in commercially available natto is only 20 FU/g to 40 FU/g. In contrast, the nattokinase activity per unit amount of the Bacillus natto culture extract is much higher than that of commercially available natto. Therefore, the advanced glycation endproduct production inhibitor according to the present invention containing the Bacillus natto culture extract can have high nattokinase activity that cannot be obtained by using commercially available natto.

Usually, when Bacillus natto are cultured, a culture medium contains both nattokinase, which is an active factor in the thrombolytic system, and vitamin K, which is an active factor in the thrombosis system. It is known that such a vitamin K counteracts the action and effect of nattokinase, and is a component that preferably needs to be removed in the present invention. Therefore, in the present invention, the content of vitamin K2 contained in the Bacillus natto culture extract is adjusted to be preferably 1 µg or less, and more preferably 0.1 µg or less, per gram of the dry mass of the Bacillus natto culture extract.

Vitamin K2 contained in the Bacillus natto culture extract can be removed by, for example, mixing a Bacillus natto culture medium containing vitamin K2 with an aqueous chitosan solution such that vitamin K2 is adsorbed onto the chitosan, and then filtering off the chitosan on which the vitamin K2 is adsorbed, as described in JP 2006-325597A, for example.

The advanced glycation endproduct production inhibitor of the present invention may contain an oily substance for the purpose of increasing the dispersibility of the Bacillus natto culture extract. The oily substance does not solidify at a temperature of, for example, preferably 50°C or lower, or more preferably 40°C or lower. Examples of the oily substances include soybean oil, glycerin fatty acid esters, beeswax, rapeseed oil, jojoba oil, palm oil, and coconut oil, and combinations thereof.

There is no particular limitation on the content of the oily substance in the advanced glycation endproduct production inhibitor of the present invention. For example, an appropriate amount can be selected by one skilled in the art based on the nattokinase activity or the like of the Bacillus natto culture extract used in combination.

The advanced glycation endproduct production inhibitor of the present invention may also contain other additives in addition to the Bacillus natto culture extract. Examples of such other additives are additives that can be typically used in the food and/or pharmaceutical fields, and specific examples thereof include excipients, stabilizers, lubricants, binders, fluidizers, disintegrants, sweetening agents, flavors, and colorants, and combinations thereof.

Examples of the excipients include erythritol, sorbitol, xylitol, maltitol, lactose, white sugar, trehalose hydrate, reduced maltose syrup, crystalline cellulose, dextrin, corn starch, potato starch, wheat starch, rice starch, partially pregelatinized starch, sodium bicarbonate, anhydrous calcium phosphate, calcium hydrogen phosphate hydrate, tribasic calcium phosphate, calcium carbonate, precipitated calcium carbonate, calcium silicate, and calcium lactate, and combinations thereof.

Examples of the stabilizers include dextrin, α-cyclodextrin, β-cyclodextrin, γ-cyclodextrin, sucrose fatty acid esters, hydroxypropyl-8-cyclodextrin, hydroxypropyl cyclodextrin, and mannitol, and combinations thereof.

Examples of the lubricants include stearic acid, magnesium stearate, calcium stearate, sodium stearyl fumarate, sucrose fatty acid esters, hydrogenated oil, glycerin, glycerin fatty acid esters, carnauba wax, and talc, and combinations thereof.

Examples of the binders include pullulan, pectin, sodium alginate, gum arabic, guar gum, agar, starch syrup, hydroxypropyl cellulose, pregelatinized starch, polyvinylpyrrolidone, carboxyvinyl polymer, polyvinyl alcohol, ammonioalkyl methacrylate copolymer, ethyl cellulose, carboxyvinyl polymer, carboxymethyl ethyl cellulose, hypromellose, polyvinyl alcohol-polyethylene glycol graft copolymer, polyvinyl alcohol-acrylic acid-methyl methacrylate copolymer, methylcellulose, beeswax, macrogol, and methacrylic acid copolymer, and combinations thereof.

Examples of the fluidizers include silicon dioxide, light anhydrous silicic acid, magnesium aluminometasilicate, and fumed silica, and combinations thereof.

Examples of the disintegrants include hydroxypropyl cellulose, carboxymethyl cellulose, carmellose calcium, corn starch, partially pregelatinized starch, sodium croscarmellose, sodium carboxymethyl starch, and crospovidone, and combinations thereof.

Examples of the sweetening agents include aspartame, saccharin, stevia, powdered sweet hydrangea leaf, sucralose, acesulfame potassium, white sugar, sorbitol, reduced maltose syrup, saccharin, liquorice, and thaumatin, and combinations thereof.

Examples of the flavors include orange, vanilla, strawberry, yogurt, menthol, fennel oil, cinnamon oil, spruce oil, peppermint oil, and green tea powder, and combinations thereof.

Examples of the colorants include iron sesquioxide, yellow iron oxide, black iron oxide, titanium oxide, talc, food yellow no. 4, food yellow no. 4 aluminum lake, food yellow no. 5, food red no. 2, food red no. 3, food red no. 102, food blue no. 1, methylene blue, carmine, and riboflavin, and combinations thereof.

There is no particular limitation on the contents of the additives in the advanced glycation endproduct production inhibitor of the present invention. For example, an appropriate amount can be selected by one skilled in the art based on the nattokinase activity or the like of the Bacillus natto culture extract used in combination.

The dosage of the advanced glycation endproduct production inhibitor is preferably 1000 to 4000 FU (fibrin degradation units), and more preferably 2000 to 4000 FU, per day for an adult (with a body weight of 60 kg to 65 kg). The advanced glycation endproduct production inhibitor may be divided and administered into several times a day.

The advanced glycation endproduct production inhibitor of the present invention is, for example, an oral formulation that can be ingested by animals such as humans, pets, livestock, poultry, and farmed fish, and is used, for example, as the food, drink, feed, and pharmaceutical products themselves or as one of the components thereof.

The advanced glycation endproduct production inhibitor of the present invention may have any dosage form, and examples of the dosage form include powdered medicine, granular medicine, pills, tablets, and liquid medicine. When such an advanced glycation endproduct production inhibitor is used as a food or drink, examples of the food or drink include general diets; supplements; foods with health claims, such as foods for specified health uses, foods with nutrient function claims, and foods with functional claims; soft drinks; tea drinks; coffee drinks; processed milk; milk drinks; soy milk; and alcoholic beverages.

The advanced glycation endproduct production inhibitor of the present invention contains, as an active ingredient, a Bacillus natto culture extract that can be obtained from natto useful as a general diet as mentioned above. For this reason, safety for ingestion by living organisms (e.g., humans) is fully guaranteed. In addition, the advanced glycation endproduct production inhibitor can be easily ingested by humans of a wide range of ages, from infants to adults and the elderly.

The advanced glycation endproduct production inhibitor of the present invention can inhibit, for example, the production of AGEs in vivo, thus making it possible to inhibit the onset and progression of, for example, type 2 diabetes and the like. Alternatively, the advanced glycation endproduct production inhibitor of the present invention can also be used as a blood AGEs concentration lowering agent for lowering the concentration of AGEs in the blood when it is ingested by humans, for example.

### Examples

Hereinafter, the present invention will be described in greater detail using examples. However, the present invention is not limited to the examples below.

### (Example 1: Production of Low-Dose Nattokinase-Containing Feed (NK-L))

NSK-SD manufactured by Japan Bio Science Laboratory Co., Ltd. was received as a Bacillus natto culture extract containing nattokinase (NK). This product is powder (containing dextrin as a stabilizer) that is obtained by filtering a culture extract obtained from a fermentation culture of Bacillus natto to remove impurities with a molecular weight of 20,000 or less, and then spray-drying the resulting filtrate, and the product contains nattokinase in an amount of 3.4% by mass. Also, the above product had a nattokinase activity of 20,000 FU/g.

Then, the preparation of feed containing 0.6% of the above-mentioned Bacillus natto culture extract (NSK-SD) per gram of normal solid feed (CE-2 manufactured by CLEAJapan, Inc.) was entrusted to CLEAJapan, Inc., to obtain a low-dose nattokinase-containing feed (NK-L) (advanced glycation endproduct production inhibitor) containing 0.2 mg of nattokinase (NK) per gram of feed.

### (Example 2: Production of High-Dose Nattokinase-Containing Feed (NK-H))

A high-dose nattokinase-containing feed (NK-H) (advanced glycation endproduct production inhibitor) containing 0.6 mg of nattokinase (NK) per gram of feed was obtained in the same manner as in Example 1, except that the preparation of feed containing 1.8% of the above-mentioned Bacillus natto culture extract (NSK-SD) per gram of normal solid feed (CE-2 manufactured by CLEAJapan, Inc.) was entrusted to CLEA Japan, Inc.

### (Example 3: Creation of STZ-Induced Type 2 Diabetes Model Animals and Administration of Nattokinase-Containing Feeds)

The following animals and reagents were used to evaluate the low-dose nattokinase-containing feed (NK-L) and the high-dose nattokinase-containing feed (NK-H) obtained in Examples 1 and 2.

### (1) Animals

All experimental procedures were carried out in compliance with the laws and regulations concerning experiments, such as the "Act on Welfare and Management of Animals", and the Hiroshima International University Experimental Animal Guidelines, and animal experiments were conducted appropriately with ethical considerations.

Male Six-week-old Spragur-Dawly (SD) rats were purchased from Japan SLC Co., Ltd. and housed in a breeding room (Hiroshima International University Kure Campus Animal Breeding Room) under conditions: a 12-hour light/dark cycle (light was from 8:00 to 20:00 and dark was from 20:00 to 8:00) at a room temperature of 23°C ± 1°C and a humidity of 55% ± 5%, with a breeding density of three rats per cage, and were allowed to acclimate for 10 days. During the acclimation period, animals that had grown to a body weight of 240 to 280 g were used in this experiment. During the acclimation period, the animals were fed normal solid feed (CE-2 manufactured by CLEA Japan, Inc.) and given free access to water using an automatic water supply valve.

### (2) Reagents

Streptozotocin (STZ), diethyl ether (special grade), a sodium citrate buffer solution with a pH of 4.5, cholesterol E-Test Wako, and LabAssay^{™} Triglyceride were purchased from Fujifilm Wako Pure Chemical Corporation and used.

Novo-Heparin 10,000 units/10 mL for Injection were purchased from Mochida Pharmaceutical Co., Ltd. and used.

A self-monitoring glucose kit diabetes sensor was purchased from Arkray, Inc. and used.

The Oxi Select TM Advanced Glycation End Product (AGE) Competitive ELISA Kit was purchased from Cosmo Bio Co., Ltd. and used.

Sureeads Protein G Magnitic Beads (1 mL) were purchased from Bio-Lad and used.

Glucose was purchased from Merck and used.

### (3) Experimental Method

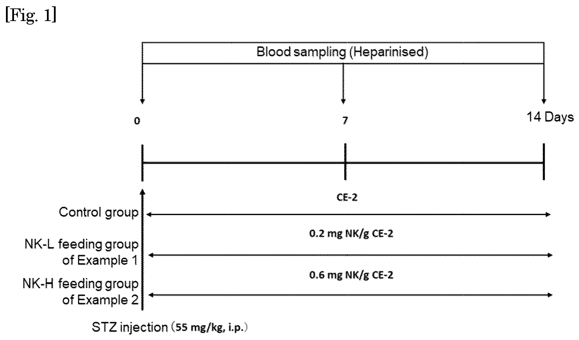

The experiment was carried out according to the design shown in FIG. 1. Specifically, the experiment was performed as follows.

Male SD rats (8 weeks old) were acclimated for 10 days and divided into three groups (n = 6) through stratified random sampling based on the two parameters of fasting blood glucose and body weight, such that the mean and variance of each parameter were uniform. Streptozocin (STZ) which was adjusted with sodium citrate to have a pH of 4.5, was administered intraperitoneally once at a dose of 55 mg/3 mL/kg body weight, and the rats were then bred for 14 days.

Immediately after the STZ administration, the control group was given normal solid feed (CE-2 manufactured by CLEA Japan, Inc.), the low-dose NK feeding group was given the low-dose nattokinase-containing feed (NK-L) (0.2 mg NK/g CE-2) obtained in Example 1, and the high-dose NK feeding group was given the high-dose nattokinase-containing feed (NK-H) (0.6 mg NK/g CE-2) obtained in Example 2, and the animals were allowed to freely ingest the feeds for 14 days.

The experiment was continued for only animals that exhibited fasting blood glucose levels of 200 mg/dL or more on Day 7 after the STZ administration and were in a diabetic state. This measure was based on the empirical rule that in individuals whose blood glucose levels did not reach abnormal levels on Day 7 after the STZ administration, no increase in blood glucose levels was observed until the end of the experiment. Breeding was performed in stainless steel rabbit cages at a breeding density of 3 rats/cage. The food intake during the experimental period was measured for each cage three times a week.

Blood glucose levels were measured and blood samples were collected immediately before the STZ administration, and on Day 7 and on Day 14 after the start of test substance administration. Immediately before the STZ administration, on Day 7 and on Day 14 (final day of the test) after the start of test substance administration, 500 µL of blood (heparinized) was collected from the tail vein of the rats that had been made to fast for 15 hours (19:00 to 10:00) from the previous day, and blood glucose levels were measured using a self-monitoring glucose meter (GT-1670, manufactured by Arkray, Inc.) using the blood (30 µL) flowing out before hemostasis as a sample. The heparinized blood was centrifuged (1500 ×g, 10 minutes) in a high-speed refrigerated microcentrifuge (MX-100 manufactured by Tomy Seiko Co., Ltd.), and the obtained plasma was stored at -80°C until the plasma was used as a sample for measuring blood biochemical parameters.

Immediately after blood collection, the animals were euthanized through exsanguination by cutting the abdominal aorta, after which the left kidney was removed and fixed in 10% formalin buffer (pH 6.8). In addition, male SD rats (8 weeks old) were kept under normal conditions for 14 days, and the left kidneys were excised in the same manner as above to prepare normal tissue preparations.

### (4) Measurement of Blood Biochemical Markers

AGEs were measured using the plasma obtained in the experiment in (4) above as a sample.

The Oxi Select TM Advanced Glycation End Product (AGE) Competitive ELISA Kit was used to measure AGEs.

### (5) Kidney Histopathological Evaluation

On Day 14 after the STZ administration, the left kidney of each individual in each group was excised, dehydrated using an automatic processor, and embedded in paraffin. Tissue preparations (4 µm) were stained with hematoxylin-eosin (HE), periodic acid-methenamine silver (PAM), and periodic acid-Schiff (PAS) to observe renal corpuscles and renal tubules.

### (6) Results

### (Body Weight and Food Intake)

Table 1 shows the body weight gain and food intake of the control group, the low-dose NK feeding group that was fed the low-dose nattokinase-containing feed (NK-L) obtained in Example 1 (also referred to as "NK-L feeding group of Example 1" hereinafter), and the high-dose NK feeding group that was fed the high-dose nattokinase-containing feed (NK-H) obtained in Example 2 (also referred to as "NK-H feeding group of Example 2" hereinafter).

**[Table 1]**

| | Control group | NK-L feeding group of Example 1 | NK-H feeding group of Example 2 |
|---|---|---|---|
| Body weight gain (g/day) | 2.0±0.37 | 2.0±0.18 | 2.0±0.24 |
| Food intake (g/day) | 31.3±0.96 | 34.1±0.97 | 32.2±1.2 |

As shown in Table 1, the average body weight gain during 14 days from the start of test substance administration was approximately 2 g/day in all groups, with no difference between the groups. There was no difference in food intake.

### (Blood Biochemical Data)

Table 2 shows the blood glucose levels of the normal group, the control group, the NK-L feeding group of Example 1, and the NK-H feeding group of Example 2.

**[Table 2]**

| | Normal value group | Control group | NK-L feeding group of Example 1 | NK-H feeding group of Example 2 |
|---|---|---|---|---|
| Glucose (mg/dL) | 67.11±1.3 | 326.75±17.0# | 419±23.3# | 401±31.0# |

| | | | | |
|---|---|---|---|---|
| #:P<0.05 | | | | |

As shown in Table 2, the blood glucose levels in all groups were 200 mg/dL or higher on Day 7 after the STZ administration (on Day 7 after the start of test substance administration), and on Day 14, the blood glucose levels rose to 326.75 ± 17.0 mg/dL in the control group, 419.8 ± 23.3 mg/dL in the NK-L feeding group of Example 1, and 401 ± 31.0 mg/dL in the NK-H feeding group of Example 2, which were significantly higher than the normal value (67.11 ± 1.3 mg/dL). On the other hand, there were no significant differences in the values between groups.

### (Histopathological Evaluation of Renal Nephrons)

The left kidney was excised from each rat in each group and the area surrounding the nephrons of the left kidney was subjected to histopathological evaluation.

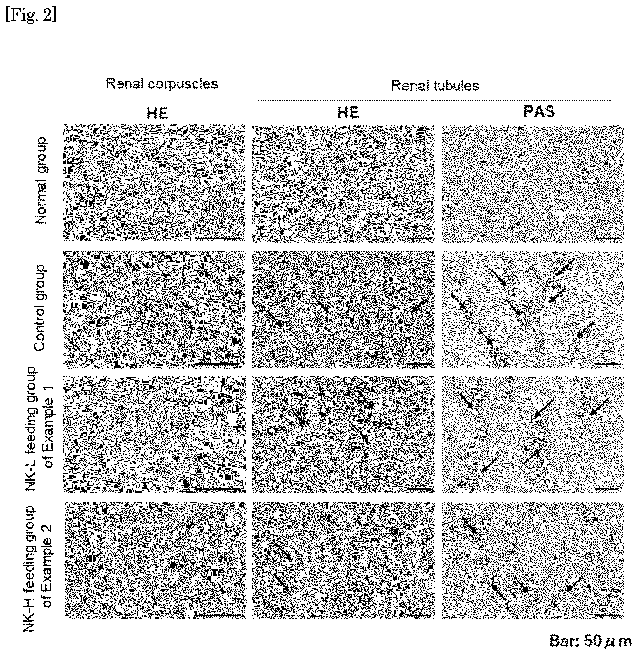

Specifically, as described in (4) Experimental Method above, the left kidney of each rat in each group was excised, dehydrated in an automatic processor, and embedded in paraffin. For the normal group (N), male SD rats (8 weeks old) were fed normal solid feed (CE-2 manufactured by CLEA Japan, Inc.) for 14 days and treated in the same manner as above. Tissue preparations were thinly sectioned (4 µm) and stained with hematoxylin-eosin (HE), periodic acid-methenamine silver (PAM), and periodic acid-Schiff (PAS) to observe renal corpuscles and renal tubules (FIG. 2).

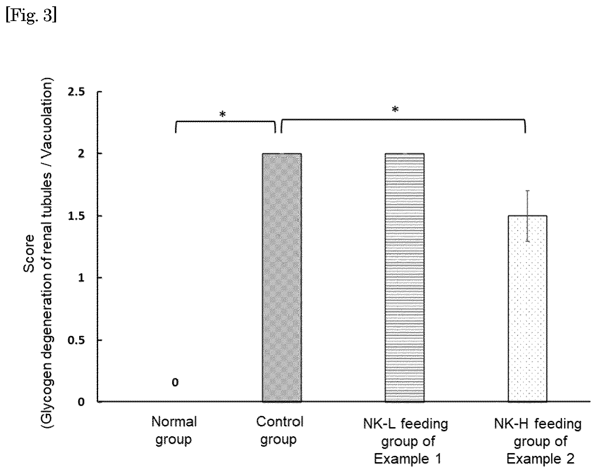

Furthermore, the glycation state of the renal tubules was scored based on epithelial cell vacuolation and positive PAS staining. Evaluation was made based on a 5-point scale (no change: -, slight change: +, moderate change: ++, sever change: +++, marked change: ++++) according to the following criteria. Testing was performed using the Mann-Whitney's U test, and the P value was calculated (*: P < 0.05). The results are shown in FIG. 3.

As shown in FIG. 2, regarding the renal corpuscles (stained with HE; left lane), in comparison with the normal group, no lesions were observed around the renal corpuscles in any of the control group, the NK-L feeding group of Example 1, the NK-H feeding group of Example 2, and no abnormalities were observed. On the other hand, regarding the renal tubules (stained with HE; center lane), vacuolation of epithelial cells was observed in all groups as indicated by the arrows, in comparison with the normal group. Regarding the renal tubules (stained with PAS; right lane), glycation of epithelial cells (positive PAS staining: glycogen deposition) was observed in all groups as indicated by the arrows, in comparison with the normal group. In addition, as shown in FIG. 3, in the control group and the NK-L feeding group of Example 1, glycation rated as ++ was observed in all individuals, whereas in the NK-H feeding group of Example 2, mild cases rated as + were observed. In the NK-H feeding group of Example 2, 3 out of 6 cases were mild cases, and their average scores were significantly lower than those of the control group.

### (Concentration of Plasma Advanced Glycation Endproducts (AGEs))

The concentrations of the plasma advanced glycation endproducts (AGEs) of the normal group, the control group, the NK-L feeding group of Example 1, and the NK-H feeding group of Example 2 were measured.

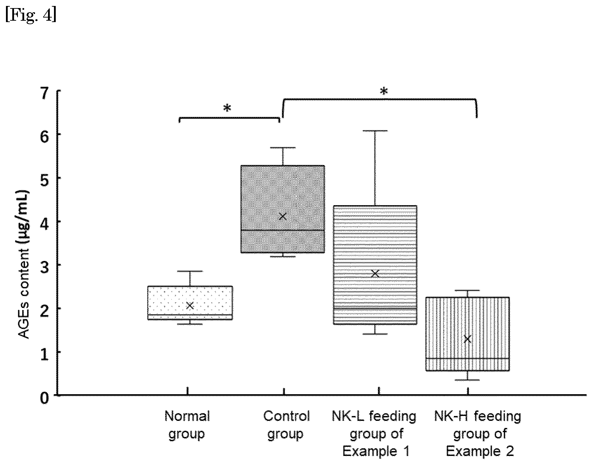

Specifically, as described in (4) Experimental Method above, measurement was made using the stored plasma samples from rats in each group, and the measured values were expressed as mean values ± standard error. Statistical analysis was performed using the Dunnet method, and the differences between the control group and the other groups were compared (*: P < 0.05). The results are shown in FIG. 4.

The concentration of the plasma AGEs obtained was 4.12 + 0.48 µg/dL in the control group, which was significantly higher than that in the normal group (2.06 ± 0.19 µg/dL). On the other hand, the concentrations of the plasma AGEs were 2.81 ± 0.75 µg/dL in the NK-L feeding group of Example 1 and 1.30 ± 0.36 µg/dL in the NK-H feeding group of Example 2, and these values decreased depending on the NK dose compared to the control group, and the NK-H feeding group of Example 2 had a significantly lower value.

### Industrial Applicability

The present invention is useful in, for example, technical fields such as the food field and the pharmaceutical field.

## Claims

1. An advanced glycation endproduct production inhibitor comprising a Bacillus natto culture extract as an active ingredient.

2. The advanced glycation endproduct production inhibitor according to claim 1, wherein the Bacillus natto culture extract contains nattokinase.

3. The advanced glycation endproduct production inhibitor according to claim 1, wherein the Bacillus natto culture extract is in the form of a dry powder.

4. The advanced glycation endproduct production inhibitor according to claim 1, wherein a content of vitamin K2 contained in the Bacillus natto culture extract is 1 µg or less per gram of a dry mass of the Bacillus natto culture extract.

5. The advanced glycation endproduct production inhibitor according to claim 1, wherein the Bacillus natto culture extract has a nattokinase activity of 100 FU/g to 80,000 FU/g.

6. The advanced glycation endproduct production inhibitor according to claim 1, wherein the advanced glycation endproduct production inhibitor is an oral formulation.
